# EUROPÄISCHE PATENTSCHRIFT

(11) **EP 1 365 727 B1**
(45) Veröffentlichungstag und Bekanntmachung des Hinweises auf die Patenterteilung: **06.03.2013**
(21) Anmeldenummer: 02724100.9
(22) Anmeldetag: 08.03.2002
(51) Int. Cl.: A61K 6/06, A61K 6/083

(54) **VERWENDUNG VON BIOAKTIVEM GLAS IN ZAHNFÜLLMATERIAL**
USE OF BIOACTIVE GLASS IN DENTAL FILLING MATERIAL
UTILISATION DE VERRE BIOACTIF DANS UN MATERIAU D'OBTURATION DENTAIRE

(30) Priorität: 09.03.2001 DE 10111449
(43) Veröffentlichungstag der Anmeldung: 03.12.2003
(73) Patentinhaber: Schott AG, 55122 Mainz (DE)
(72) Erfinder: KESSLER, Susanne, 84030 Ergolding (DE); LEE, Sean, 76227 Karlsruhe (DE)
(74) Vertreter: Fritzsche, Thomas
(86) Internationale Anmeldenummer: PCT/DE2002/000827
(87) Internationale Veröffentlichungsnummer: WO 2002/072038

(56) Entgegenhaltungen:
- EP-A- 0 634 373
- EP-A- 0 716 049
- EP-A- 0 997 132
- WO-A-97/27148
- DE-A- 19 858 126
- DE-C- 4 100 604

## Beschreibung

Die Erfindung betrifft die Herstellung von ästhetischem sich nicht verfärbenden permanenten Zahnfüllungsmaterial, welches der Entstehung von Sekundärkaries entgegenwirkt.

Bei Zahnrestaurierungen wird aus ästhetischen Gründen angestrebt, den restaurierten Zahnbereich möglichst unsichtbar erscheinen zu lassen, sodass er vom natürlichen Zahn visuell nicht zu unterscheiden ist. Dies wird bislang mit Compositen, Kompomeren und Glas-Ionomer-Zementen oder auch mit teuren Porzellan- oder Glaskeramik-Inlays zum Teil mehr oder weniger gut erreicht. Derartige Füllungen zeigen jedoch eine nur unzureichende Haltbarkeit und werden rasch ausgewaschen. Darüber hinaus neigen sie auch zur Verfärbung.

Bioaktive Gläser sind bereits seit langem bekannt und beispielsweise zusammenfassend von Larry L. Hench und John K. West in "Biological Applications of Bioactive Glasses", Life Chemistry Reports 1996, vol. 13, p. 187 - 241 oder in "An Introduction to Bioceramics", L. Hench und J. Wilson, eds. World Scientific, New Jersey (1993) beschrieben. Bioaktive Gläser zeichnen sich im Gegensatz zu herkömmlichen Gläsern dadurch aus, dass diese in einem wässrigen Medium löslich sind und an ihrer Oberfläche eine Hydroxylapatitschicht ausbilden. Die gängigsten bioaktiven Gläser werden entweder als Schmelzglas hergestellt, wobei diese dann gegenüber normalen Fenster- oder Flaschengläsern einen deutlich geringeren Anteil an SiO₂ und einen wesentlich höheren Anteil an Natrium aufweisen oder sie sind sogenannte Sol-Gel-Gläser, welche dann, im Gegensatz zu Schmelzgläsern einen hohen Anteil von Siliziumoxid sowie einen geringen bis gar keinen Anteil an Natrium enthalten können.

Die wesentlichen Eigenschaften von bioaktivem Glas sind dem Fachmann bekannt und beispielsweise in der US-A 5,074,916 beschrieben. Danach unterscheidet sich bioaktives Glas von herkömmlichen Kalk-Natrium-Silikat-Gläsern dadurch, dass es lebendes Gewebe bindet.

Derartige bioaktive Gläser werden beispielweise zur Heilung von Knochenschäden, insbesondere als synthetisches Knochentransplantat verwendet. Darüber hinaus werden sie zur Heilung von chronischen Wunden, insbesondere bei diabetischen Geschwüren, sowie bei Druck- und Liegewunden in der Geriatrie mit Erfolg eingesetzt. So konnten beispielsweise John E. Rectenwald, Sean Lee und Lyle L. Moldawer et. al. (Infection and Immunity, zur Publikation eingereicht) zeigen, daß bioaktives Glas bei der Maus eine inflammatorische Wirkung zeigt, welche durch eine Stimulierung der Interleukin-6 (IL-6) - Aktivität bei gleichzeitiger Inhibierung der entzündungsstimulierenden Cytokine TNF-alpha, IL-1-alpha und IL-10 sowie MPO (Myeloperoxidase) (siehe auch 19. Annual Meeting, Surgical Infection Society 1999 28.4.-01.05.1999).

Darüber hinaus ist von E. Allen, et. al. (Departments of Microbiology in Periodontology Eastman Dental Institut) bekannt, daß ein bioaktives Glas 45-S-5 welches von Bioglas^{®} U.S. Biomaterials Alachua, FL. 32615 USA erhältlich ist und das eine antibakterielle Wirkung zeigt. Eine solche Wirkung kann mit normalen Glaskügelchen, sog. Glasbeads (Fensterglas) nicht erreicht werden.

In der WO 97/27148 wird vorgeschlagen, bioaktives Glas einer definierten Zusammensetzung zur Remineralisierung von Zähnen zu verwenden. Das bioaktive Glas besteht aus Pulver mit zwei verschiedenen Teilchengrößen, nämlich solchen mit einer durchschnittlichen Teilchengröße von höchstens 90 µm und solchen von höchstens 10 µm. Zur Remineralisierung setzt das bioaktive Glas durch Ionenaustausch zahnmedizinisch wichtige Mineralien frei, wobei die großen Teilchen als Ionenreservoir dienen sollen. Dadurch wird neues Apatit, insbesondere Hydroxylapatit, ausgebildet, das vom natürlichen Hydroxyl-Apatit des Zahns nicht zu unterscheiden ist. Darüber hinaus hat das bioaktive Glas zugleich eine desensibilisierende sowie eine antibakterielle Wirkung. Die kleinen Partikel sollen in feine Zahndefekte wie z. B. Fissuren eindringen, diese verschließen und überziehen. Insbesonders durch mehrfache Applikation sollen auf diese Weise Zahndefekte versiegelt werden.

Aus der US 5,891,233 ist die Verwendung eines bioaktives Glas enthaltenden Materials als Unterfutter für eine temporäre Zahnfüllung bekannt. Darin hat das bioaktive Glas die Aufgabe, Dentinkanälchen zu remineralisieren und Zahnirritationen und Schmerzen zu vermeiden. Ferner wird die Verwendung eines Materials, welches hauptsächlich aus bioaktivem Glas besteht, als temporäre Füllung des Wurzelkanals vorgeschlagen, um diesen zu festigen, bevor sie später durch eine endgültige Füllung ersetzt wird. Auch wird dort bereits vorgeschlagen, mit diesem Material überkronte Prothesen temporär zu befestigen bevor diese endgültig überkront werden.

In der DE 198 14 133 werden selbstdesinfizierende Kunststoffe für den Dentalbereüch beschrieben, die eine verringerte Adhäsion von Mikroorganismen aufweisen und die biozide Substanzen enthalten, welche die Mikroorganismen innerhalb 24 Stunden abtöten. Als biozide Substanzen werden Silber, Kupfer und Zink, sowie organische Verbindungen wie Ciprofloxalin, Chlorhexidin und andere beschrieben.

Weiterhin wird in EP 0 716 049 eine Verwendung von bariumfreien Glaspartikeln zur Herstellung eines Mittels für eine Zahnfüllung vorgeschlagen, wobei die Glaspartikel eine mittlere Teilchengröße von <10 *µ*m aufweisen.

Zudem wird in EP 0 997 132 ein bariumfreies, röntgenopakes Dentalgas-Kunststoff-Komposit mit einem Dentalglasgehalt von bis zu 80 Gew.% als Zahnfüllung offenbart. Das Glaspulver hat eine mittlere Teilchengröße von 10 *µ*m.

Auch in EP 0 997 132 wird ein bariumfreies Dentalglas mit einer Teilchengröße von 10 *µ*m offenbart.

In der deutschen Anmeldung DE 198 58 126 A wird ein Glaspulver für einen dentalen Glasionomerzement für Zahnfüllungen mit einer Teilchengröße von 0,2-4 *µ*m beschrieben.

In der WO99/07326 wird eine antimikrobiell wirksame Zementzusammensetzung beschrieben, welche im medizinischen Bereich (Knochenzement, Implantantatkomponenten) und im Dentalbereich (Füllmaterialien, Adhesive, Versiegelungsmittels und restoratives Material) Verwendung finden soll. Dabei sollen die Nachteile überwunden werden, die durch das Freisetzen von Fluoridionen aus Glasionomerzementen nach dem Stand der Technik entstehen. Durch das Fluorid soll ein antimikrobieller Effekt erreicht werden. Das Freisetzen von Fluorid aus der Glasmatrix und dessen Ersatz durch andere Ionen führt nämlich zu einer strukturellen Veränderung sowohl des Zementes als auch der Kavität was vermieden werden soll. Darüber hinaus wird durch die Freisetzung der Fluoridgehalt im Matrixmaterial erschöpft, so daß dieses Material regelmäßig regeneriert werden muß, um einen kontinuierlichen Effekt gegen schädliche Bakterien zu erzielen. Aus diesem Grund wird zur Erzielung eines antimikrobiellen Effektes vorgeschlagen, dem Zahnfüllmaterial ein antimikrobiell wirkendes Zeolith zuzusetzen. Es wird jedoch auch beschrieben, daß derartige Zeolithe eine Verfärbung des Polymermaterials bewirken, weshalb diese nicht farbstabil sind. Das eingesetzte Zeolith soll über die Zeit biozide Metallionen, wie beispielsweise Silberionen, freisetzen.

Eine dauerhafte Verwendung von bioaktivem Glas ist bislang nicht beschrieben, da davon auszugehen war, dass sich dieses durch den Kontakt mit Körperflüssigkeit unter Freigabe von Ca, Na und P anlöst und porös wird. Daher wird das Material bisher nur als temporäres Zahnfüllmaterial verwendet.

Die Erfindung hat zum Ziel, ein Zahnfüllmaterial bereitzustellen, das nicht nur temporär sondern dauerhaft im Zahn verbleibt, das im Wesentlichen unsichtbar ist, und sich nicht mit der Zeit verfärbt. Darüber hinaus soll es die Nachteile des Standes der Technik nicht aufweisen und insbesonders die Ausbildung von Sekundärkaries vermeiden. Schließlich soll es auch eine hohe mechanische Belastbarkeit und Härte zeigen, die Schmerzempfindlichkeit beseitigen und antimikrobiell wirken. Erfindungsgemäß werden diese Ziele durch die in den Ansprüchen definierten Merkmale erreicht.

Es wurde nämlich überraschenderweise gefunden, daß sich durch eine Kombination von bioaktivem Glas und herkömmlichem Dentalglas Mischungen herstellen lassen, die, eingebunden in eine Matrix, nicht sichtbar sind und sich vom natürlichen Zahnmaterial nicht unterscheiden. Dabei wird erfindungsgemäß derart vorgegangen, daß zuerst der Brechungsindex des Matrixmaterials bestimmt wird und dann ein herkömmliches Dentalglas ausgewählt bzw. zugemischt wird, dass den gleichen oder annähernd den gleichen Brechungsindex wie die Matrix aufweist. Unter "annähernd den gleichen Brechungsindex" sind sämtliche Brechungsindices zu verstehen, welche nicht mehr visuell erkennbar sind. Es hat sich gezeigt, dass der Brechungsindex von bioaktivem Glas dann vernachlässigbar ist, wenn die Brechungsindices beim Dentalglas und bei der Matrix im wesentlichen gleich sind. Dies trifft insbesondere auf die bevorzugten Mengenanteile zu. Derartige Mischungen bzw. Kits, welche die Komponenten in entsprechenden Anteilen enthalten, lassen sich bequem vom Hersteller vorkonfektionieren, sodass der Zahnarzt die Komponenten nur noch zusammenzumischen braucht.

Auf diese Weise ist es möglich, äußerst ästhetisch wirkende Zahnfüllmaterialien, insbesondere für den sichtbaren Bereich, bereitzustellen, die mechanisch beanspruchbar und hart sind, und die sich auch mit der Zeit nicht verfärben. Darüber hinaus zeigen derartige Füllmaterialien hervorragende Eigenschaften zur Vermeidung von Schmerzen und zum Schutz des gesunden Zahnmaterials.

Da nämlich bioaktives Glas löslich ist, war zu erwarten, dass es sich beim Zusammentreffen mit wasserhaltigen Körperflüssigkeiten wie Speichel oder mit Getränken soweit auflöst, dass es entweder als Gel vorliegt oder es derart porös wird, dass es bei der geringsten Belastung zerbricht. Durch den Eintrag eines bioaktiven Glases in das Zahnfüllmaterial ist daher zu erwarten gewesen, dass es nach dem Auflösen neue Kavitäten und ein Hohlnetzwerk in der Zahnfüllung zurück läßt, welche die Festigkeit der Füllung beeinträchtigt.

Darüber hinaus werden moderne Zahnfüllungen üblicherweise mittels Kunststoffklebern im ausgebohrten Hohlraum befestigt. Damit diese Kunststoffkleber ausreichend halten, ist es notwendig, das im Hohlraum vorliegende natürliche Dentin zuerst mittels einer Säure anzuätzen um eine rauhere Oberfläche zu erhalten an der das eientliche Klebematerial besser hält. Hierzu wird vorzugsweise Phosphorsäure und eine organische Säure, wie z.B. Methacrylsäure verwendet. In modernen Zahnfülltechniken wird dies jedoch in einem einzigen Schritt durchgeführt, d.h. es werden Klebematerialien bzw. sogenannte Bondings verwendet, welche die entsprechenden Säuren bereits zugemischt enthalten und dabei während des Abbindens das Zahnmaterial anätzen. Da bioaktive Gläser einen außergewöhnlich hohen pH-Wert haben, ist zu erwarten, dass Zahnfüllmaterialien, welche diese Gläser enthalten, das Bonding sofort neutralisieren oder dessen pH-Wert sogar ins alkalische verschieben, wodurch der Ätzeffekt verhindert wird. Dadurch ist zu erwarten, dass der Klebeeffekt, d.h. die Klebekraft vermindert wird. Dies ist bei temporären Füllungen weniger problematisch, jedoch für den Einsatz als permanente Dauerfüllung müssen derartige Klebestellen im Zahngut besonders haltbar verankert sein.

Es wurde nun überraschenderweise gefunden, dass die durch Anlösung des Glasmaterials erzeugten Kavitäten die innere Haltbarkeit der Füllung insgesamt nicht oder nur in vernachlässigbarem Umfang beeinträchtigen. Auch die durch Anätzen mit Säuren erhöhte Haftfestigkeit der Bondings wird durch die stark alkalischen Biogläser überraschenderweise nicht vermindert.

Eine deutliche Anlösung der bioaktiven Glaspartikel findet nur an der Oberfläche der Füllung statt. Es wurde nun auch gefunden, dass diese so gering ist, dass sie nicht oder nur unwesentlich, d.h. vernachlässigbar größer ist als die gleichzeitig ablaufende natürliche Abrasion des Zahnes und des Füllungsmaterials, die ca. 10 *µ*m pro Jahr beträgt. Der zeitliche Verlauf dieser Löslichkeit ist gesteuert von der Diffusion der Ca-, Na- und Phosphat-Ionen aus dem Glas heraus.

Im Inneren der Füllung kommt als geschwindigkeitsbestimmender Schritt die Diffusion von Feuchtigkeit durch die Matrix und die Diffusion der Ca- und Na-Ionen durch die Matrix hinzu.

Bei den bislang verwendeten Dentalgläsern geringer Löslichkeit werden mit der Zeit einzelne, an der Oberfläche liegende Glaspartikel als Ganzes herausgelöst wodurch in der Oberfläche der Füllung offene Poren entstehen, in die sich Mikroorganismen einnisten können und die auch sonst eine weitere Angriffsfläche für mechanische abrasiven Abbau bieten.

Bei bioaktivem Glas wird nun gefunden, dass der obige Mechanismus nicht auftritt, sondern dass aufgrund der geringen Härte, ein anderer Mechanismus abläuft. Wird nämlich ein solcher Glaspartikel an der Oberfläche der Füllung freigelegt, wird er vom Speichel angelöst und setzt Na-, Ca-, P-Ionen frei, mit denen die Zähne und insbesondere das die Füllung übergebende Zahnmaterial, wieder remineralisiert werden. Das bioaktive Glas dient dabei sowohl als dauerhaftes Antikariesmittel sowie als dauerhaftes Desensibilisierungsmittel bei schmerzempfindlichen Zähnen gegenüber Hitze, Kälte, Säure und Süßem. Durch das permanente Freisetzen von remineralisierenden und damit zahnschützenden Ionen entsteht an der Oberfläche des Glaspartikels eine vergleichsweise weiche Silikagelschicht sowie eine Hydroxylapatitschicht, die durch Kaubewegungen langsam abgetragen wird. Dabei wird das Glas in etwa im gleichen Maße abgetragen, wie die Harzmatrix der Füllung. Die von den üblichen Dentalgläsern bekannten, offenen Poren an der Oberfläche der Füllung entstehen dabei also nicht.

Erfindungsgemäß wird daher vorgeschlagen, bioaktives Glas enthaltendes Material auch als permanente Zahnfüllung zu verwenden.

Hierdurch lassen sich die günstigen Eigenschaften des bioaktiven Glasmaterials auch für permanente Zahnfüllungen nutzen, insbesondere dessen Fähigkeit zur Apatitbildung und Desensibilisierung von Zahnirritationen, sowie dessen dauerhaft bakteriostatischen, insbesondere kariostatischen Wirkungen.

Zum Tragen kommt die bakteriostatische Wirkung und die Remineralisierung an der Kaufläche und in der ersten Zeit nach dem Legen der Füllung in den eventuell durch Polymerisationsschrumpf (bei ungenügendem Bonding) entstehenden Randspalten, aber auch direkt an der Oberfläche der Kavität.

Die erfindungsgemäß enthaltenen bioaktiven Gläser sind vorzugsweise ein herkömmliches bioaktives Glas, welches dem Fachmann bestens bekannt ist. Solche Gläser enthalten üblicherweise maximal 60 Gew.% SiO₂, einen hohen Anteil an Na₂O und CaO sowie Phosphor und zwar in einem hohen Molverhältnis von Calcium zu Phosphor, welches sich meist, jedoch nicht notwendigerweise um etwa 5 bewegt. Kommen solche bioaktiven Gläser mit Wasser oder einer Körperflüssigkeit in Kontakt, dann zeichnen sie sich durch spezielle Reaktionen aus, und zwar werden dabei Natrium- und Calciumionen des Glases durch H⁺-Ionen aus der Lösung in Form einer Kationen-Austauschreaktion ersetzt, wodurch eine Silanol-Gruppen aufweisende Oberfläche entsteht, an welche sich Natrium- und Calciumhydroxid anlagern. Die Erhöhung der Hydroxy-Ionenkonzentration führt an der Glasoberfläche nun zu einer weiteren Reaktion mit dem Siliziumnetzwerk, wodurch weitere Silanolgruppen entstehen, die auch tiefer im Glas liegen können.

Aufgrund des hohen alkalischen pH im Glaszwischenraum entsteht eine gemischte Hydroxylapatit-Phase aus CaO und P₂O₅, welche auf der SiO₂-Oberfläche auskristallisiert und in biologischen Materialien mit Mucopolysacchariden, Kollagenen und Glycoproteinen bindet.

Das Molverhältnis von Calcium zu Phosphor ist vorzugsweise > 2 und insbesondere > 3 und ist vorzugsweise < 30, insbesondere < 20, wobei Verhältnisse von < 10 besonders bevorzugt sind.

Besonders bevorzugt sind Zahnfüllmaterialien, die bioaktive Glaspartikel enthalten, welche SiO₂, CaO, Na₂O, P₂O₅, CaF₂, B₂O₃, K₂O, und/oder MgO aufweisen. Enthält das Zahnfüllmaterial bioaktive Glaspartikel aus Schmelzglas, dann weisen diese vorzugsweise bezogen auf das Gesamtgewicht an Glas von 35 - 60, vorzugsweise 35 - 55 Gew.% SiO₂, 10 - 35, vorzugsweise 15 - 35 Gew.% CaO, 10 - 35, vorzugsweise 15 - 35 Gew.% Na₂O, 1 - 12, vorzugsweise 2 - 8 Gew.% P₂O₅, 0 - 25 Gew.% CaF₂, 0 - 10 Gew.% B₂O₃, 0 - 8 Gew.% K₂O, und oder 0 - 5 Gew.% MgO auf. Ist das bioaktive Glas ein Schmelzglas, dann liegt die Obergrenze an enthaltendem Siliziumdioxid bei 60 vorzugsweise bei 55 Gew.%, wobei eine Obergrenze von 50 Gew.% besonders bevorzugt ist. Der Gehalt an Natriumoxid beträgt vorzugsweise mehr als 15 Gew.%, insbesondere mehr als 18 Gew.%. Ein Natriumoxid-Gehalt von > 20 Gew.% ist besonders bevorzugt.

Ist das im erfindungsgemäßen Zahnfüllmaterial enthaltene bioaktive Glas ein mittels Sol-Gel-Verfahren hergestelltes bioaktives Glas, dann kann sein Anteil an Siliziumdioxid bedeutend höher liegen als bei Schmelzgläsern und sein Anteil an Natriumoxid gleich 0 sein. Mit einem Sol-Gel-Verfahren hergestellte bioaktive Gläser enthalten vorzugsweise 40 bis 90 Gew.% SiO₂, 4 bis 45 Gew.% CaO, 0 bis 10 Gew.% Na₂O, 2 bis 16 Gew.% P₂O₅, 0 bis 25 Gew.% CaF₂, 0 bis 4 Gew.% B₂O₃, 0 bis 8 Gew.% K₂O und/oder 0 bis 5 Gew.% MgO.

Der Gehalt an Phosphoroxid beträgt bei beiden der zuvor beschriebenen Arten von bioaktiven Gläsern vorzugsweise mindestens 2 Gew.%, insbesondere mindestens 4 Gew.%.

Die Glasteilchen sind in den erfindungsgemäßen Zahnfüllungen in einer durchschnittlichen Korngröße d₅₀ < 50 *µ*m, insbesondere von < 20 *µ*m bzw. < 10 *µ*m enthalten, wobei Partikelgrößen von < 5 *µ*m besonders bevorzugt sind. Prinzipiell bewirkt ein höheres Verhältnis von Oberfläche zu Gewicht bzw. Volumen eine höhere sterilisierende biozide Wirkung als bei größeren Partikeln. Eine besonders hohe biozide Wirkung des bioaktiven Glases wird beispielsweise mit Teilchen in durchschnittlichen Größen von d₅₀ < 2, insbesondere < 1 *µ*m erreicht. Dabei geben die Körnungsangaben d₅₀ bzw. d₉₉ den Äquivalentdurchmesser an, bei dem die Verteilungssumme der Teilchen den Wert 50% bzw. 99% annimmt. Die Körner für die verwendeten Glasteilchen, d. h. sowohl für die herkömmlichen Dentalgläser als auch die Biogläser sind vorzugsweise gleich bzw. liegen im gleichen Bereich. Dabei ist die Korngrößenverteilung derart, daß der Wert d₉₉ das maximal zehnfache, vorzugsweise maximal achtfache, insbesondere das siebenfache des d₅₀-Wertes beträgt. Bevorzugt sind Kornverteilungen mit einem d₉₉-Wert, der maximal ca. das fünffache des d₅₀-Wertes beträgt. Damit betragen die für die erfindungsgemäße Verwendung üblichen d₉₉-Werte maximal 100 *µ*m, vorzugsweise maximal 50 *µ*m, insbesondere maximal 30 *µ*m. In speziellen Fällen betragen die Korngrößen für den d₉₉-Wert maximal 20 *µ*m. Ganz besonders bevorzugt sind Körner mit geringem Mahlabrieb.

Übliche Refraktionsindices n_{d} für Dentalgläser betragen erfindungsgemäß 1,45 bis 1,9 und vorzugsweise 1,49 bis 1,8. Die Obergrenzen der erfindungsgemäß eingesetzten Dentalgläser betragen üblicherweise 1,9, vorzugsweise 1,8 und insbesondere maximal 1,65 wobei die unteren Grenzen bei 1,45, insbesondere 1,48 und üblicherweise bei 1,49 liegen. Der klassische Bereich für derartige Gläser liegt bei 1,50 bis 1,60 insbesondere bei 1,56. Herkömmliche Dentalgläser sind generell bekannt und sind beispielsweise von Schott Glas, Landshut, Deutschland, kommerziell erhältlich.

Beträgt der Anteil an Bioglas weniger als 20 Gew.-%, insbesonders weniger als 10 Gew.-% bezogen auf den Gesamtgehalt an Glaskörpern, dann hat es sich gezeigt, daß es möglich ist, dessen Brechungsindex zu vernachlässigen und ein Dentalglas zu verwenden, dessen Brechungsindex gleich oder annähernd gleich desjenigen der Matrix ist, d. h. der visuell nicht unterscheidbar ist bzw. vernachlässigbar ist.

Durch eine Silanisierung ist es möglich, die erfindungsgemäß verwendeten Glasteilchen insbesondere in eine Kunststoffmatrix besser einzubinden, was zu einer Erhöhung der mechanischen Belastungswerte wie Biege- und Druckfestigkeit sowie der Vickershärte führt. Übliche Silanisierungsmittel sind dem Fachmann bekannt, wobei 3-Methacryloyl-oxipropyltrimethoxisilan nur beispielhaft erwähnt werden soll.

Der Gesamtanteil der Gläser am Zahnfüllmaterial beträgt üblicherweise maximal 87% bzw. 85%, wobei maximal 80%, insbesondere maximal 70% bevorzugt ist. In einigen Fällen haben sich Maximalwerte von 70%, insbesondere 65%, als ausreichend erwiesen. Erfindungsgemäß beträgt der Anteil an bioaktivem Glas am Gesamtglas maximal 50%, wobei maximal 40%, insbesondere maximal 20 Gew.-% bevorzugt ist.

Das erfindungsgemäß eingesetzte Dentalglas kann sowohl ein inertes als auch ein reaktives Dentalglas sein. Inerte Dentalgläser sind dabei solche, die keine oder nur unwesentlich Ionen freisetzen. Das heißt, sie sind selbst nicht reaktiv. Derartige inerte Dentalgläser sind meist alkalifreie Gläser. Nicht inerte, d. h. reaktive Dentalgläser, sind solche, welche ionenlässig sind und die mit der Matrix, insbesondere dem Kunststoffmonomer reagieren bzw. selbst zu dessen Aushärten beitragen. Hierzu gehören die üblichen Kompomergläser bzw. Glasionomerzementgläser. Generell sind Kompomergläser und Glasionomerzemente gleich bzw. vergleichbar. Kompomergläser sind jedoch in eine Matrix eingebunden, in der sie weniger reaktiv sind und damit eine wesentlich höhere Aushärtzeit zeigen. Sie werden daher üblicherweise zusammen mit lichtaushärtenden Monomeren bzw. einem lichtaktivierten Starter verwendet. Sie sind jedoch erfindungsgemäß gegenüber reaktiven Matrices bevorzugt. Ganz besonders bevorzugt sind inerte Dentalgläser. Das erfindungsgemäß hergestellte Zahnfüllmaterial ist vorzugsweise frei von Zeolithen.

Bevorzugt beträgt der Anteil des bioaktiven Glases in der. Materialmatrix höchstens 40, zweckmäßigerweise bis zu 20 Vol.%, vorzugsweise bis zu 15 vol.% und insbesondere höchstens 10 Vol.%, wobei 2 bis 10 Vol.% und insbesondere 4 bis 6 bzw. 5 Vol.% besonders bevorzugt ist.

In dem erfindungsgemäßen Zahnfüllmaterial ist das bioaktive Glas üblicherweise in eine Kunstharzmatrix eingebunden, die zusätzlich nicht bioaktive, herkömmliche Dentalglaspartikel enthält. Bevorzugt wird das bioaktive Glas und/oder die nicht bioaktiven Dentalglasanteile silanisiert, um das Glas besser in die Kunstharzmatrix einbinden zu können. Derartige Silanisierungen sowie Kunstharzmatrices sind dem Fachmann (z.B. Introduction to Dental Materials, Richard von Noort, Mosley Verlag, UK) bekannt und beispielsweise in entsprechenden Lehrbüchern der Zahnmedizin oder in Ullmann's Encyclopedia of Industrial Chemistry, 4th electronic edition, Dental Materials, beschrieben. Man erhält dann einen bioaktives Glas enthaltenden Glas-Ionomer-Zement. An dieser Stelle soll kurz darauf hingewiesen werden, dass im folgenden - wie allgemein üblich - die Zusammensetzung des einzelnen Glases in Gew.% angegeben wird. Die Zusammensetzung von Kompositmaterialien bzw. der Zahnfüllungsmatrix wird jedoch - wie im Dentalbereich üblich - in Vol.% angeführt.

Als Bindemittel der Zahnfüllungsmatrix wird bevorzugt PMMA (Polymethylmethacrylat) und bis-GMA, ein Polymer aus Bisphenol A-, di(2,3 epoxy propyl)ether (sog. Bombenmonomere) und Acrylsäure verwendet.

In einer ganz speziellen Ausführungsform enthält die Matrix, insbesondere aber das darin enthaltene bioaktive Glas, Barium und/oder Strontium, um so die Füllung röntgenopak zu machen, gegebenenfalls enthält es auch Zink. Hierzu wird das Ca im bioaktiven Glas ganz oder teilweise durch Zink, Barium und/oder Strontium ersetzt was bereits bei der Herstellung der Glasgrundmasse oder beispielsweise durch Ionenaustausch möglich ist. Generell kann ein Röntgen-opakes Mittel auch separat dem fertigen Glaspulver und/oder anderen Bestandteilen des Zahnfüllmaterials zugesetzt werden.

In einem bevorzugten Ausführungsbeispiel eines bioaktives Glas enthaltenden Zahnfüllmaterials wird ein Glas-Kunststoff-Composit unter Verwendung von bioaktivem Glas und gegebenenfalls einem üblichen Dentalglas vorgeschlagen. Zweckmäßige Dentalkunststoffe umfassen überwiegend UVhärtbares Harz auf Acrylat-, Methacrylat-, 2,2-Bis-[4-(3-Methacryloxy-2-hydroxypropoxy)-phenyl]-propan-(bis-GMA-), Urethan-Methacrylat-, Alcandiol-dimethacrylat- oder Cyanacrylatbasis.

Wird das erfindungsgemäß bioaktives Glas enthaltende Material in Glas-Ionomer-Zement verwendet, kann es zusätzlich übliche bei Dentalmaterialien verwendete organische Säuren wie z.B. Acrylsäure, Itakonsäure, Maleinsäure, Weinsäure sowie gegebenenfalls ein übliches Glas-Ionomer-Glas enthalten.

Vorzugsweise ist der optische Brechungsindex des bioaktiven Glases angenähert gleich dem Brechungsindex des das Glas umgebenden Matrixmaterials. Hierdurch wird das Füllmaterial insgesamt klar transparent und ist dann vom natürlichen Zahnschmelz praktisch nicht mehr zu unterscheiden, was insbesondere vorteilhaft ist, wenn die Zähne aus kosmetischen Gründen weiß gebleicht sind. Vorzugsweise beträgt der Brechungsindex der Füllung 1,45 bis 1,65 und insbesondere 1,5 bis 1,6.

Erfindungsgemäß wird eine Mischung von bioaktivem Glas mit normalem Dentalglas verwendet. Diese können in einem beliebigen Mischungsverhältnis eingesetzt werden. Der Anteil, d.h. der Füllgrad von Glas an derartigen Compositmaterialien, beträgt maximal 90 Vol.% und vorzugsweise mindestens 10 Vol.%, insbesondere mindestens 15 Vol.% und besonders bevorzugt mindestens 30 Vol.%, wobei 65 bis 85 Vol.% und 70 bis 80 Vol.% ganz besonders bevorzugt sind. Hiervon beträgt der Anteil an bioaktivem Glas, d.h. der Anteil am Gesamtglas, vorzugsweise bis zu 40, insbesondere bis zu 20 Vol.%, wobei 3 bis 10, vorzugsweise 4 bis 6 Vol.% besonders bevorzugt sind.

In einer erfindungsgemäßen Ausführungsform ist dem Füllmaterial ein oder mehrere Opaker und/oder ein oder mehrere Pigmente, wie z.B. TiO₂, beigefügt. Damit ist es möglich, die Farbe der Füllung der jeweiligen Eigenfarbe des Zahns anzupassen.

Bevorzugt hat das partikelförmige bioaktive Glas eine durchschnittliche Korngröße d₅₀ von < 10 *µ*m, zweckmäßigerweise < 5 *µ*m, bevorzugt < 4*µ*m, besonders bevorzugt 0,5 bis 2 *µ*m. Hierdurch lässt sich die Oberfläche der permanenten Zahnfüllung optimal polieren, ohne dass eine Rauigkeit auftritt. Ein Verfahren zum Vermahlen von Glas auf eine derartige Partikelgröße ist in der der US-A-5,340,776 entsprechenden DE 41 00 604 C1 beschrieben.

Bevorzugt sind die Glaspartikel in pastösem Material, einer Lösung oder einer Suspension enthalten. Derartige Pasten etc. sind beispielsweise durch Suspendieren der Glaspartikel in einem Lösungsmittel erhältlich. Bevorzugte Lösungsmittel sind Wasser, Aceton, Äther, Esther sowie Mischungen und Emulsionen davon. Besonders bevorzugt umfassen die Lösungsmittel zumindest teilweise leichtflüchtige Lösungsmittel. Zweckmäßigerweise werden den Lösungsmitteln weitere bioaktive Stoffe und Substanzen, wie Mineralsalze, organische Reaktionskomponenten, Konservierungsmittel bzw. biozide, insbesondere bakteriozide Agentien zugesetzt. Ein besonders bevorzugtes Lösungsmittel ist eine physioligische Salzlösung.

Das bioaktive Glasmaterial kann ggf. weitere Oxide oder Salze von einem oder mehreren der Elemente Na, K, Ca, Mg, B, Ti, P, F oder auch andere Elemente und Substanzen in unterschiedlichen Anteilen enthalten.

Das bioaktive Glasmaterial ist erfindungsgemäß auch direkt in Bindemittel (Bonding) zur Verbindung einer Zahnfüllung mit einem Zahn einsetzbar, etwa als zähflüssige Substanz. Die Erfindung betrifft somit auch ein Bonding das bioaktives Material enthält. Dieser Grenzbereich zwischen Füllung und Zahn ist anfällig für Sekundärkaries, insbesondere durch Spaltbildung nach Polymerisationsschrumpf der Füllung. Hier kommt die antibakterielle und antikariotische Wirkung von bioaktivem Glas zum Tragen. Dieser Aspekt hat selbständige Bedeutung.

Zur Kariesvorsorge kann das bioaktive Glas Fluor enthalten. Hierzu wird beispielsweise einer der Rohstoffe für

die Schmelze anteilig als Fluorid zweckmäßigerweise in Form von Salzen zugegeben. Dabei ersetzt das Fluorid im Glas üblicherweise bis zu 20 Gew.%, zweckmäßigerweise bis zu 10 %, wobei 2 - 10 Gew.% besonders bevorzugt sind. In vielen Fällen hat sich ein Fluoridgehalt von bis zu 5 % bzw. 0,1 - 5 Gew.% und sogar bis 2 % bzw. 1 - 2 Gew.% (jeweils bezogen auf das Fluoridion) als ausreichend erwiesen. Es ist jedoch auch möglich Fluorid dem Füllmaterial zuzusetzen und zwar separat und/oder als Vorgemisch beispielsweise zum Harz bzw. Kunststoff. Die eingesetzten Gew.% sind dabei die gleichen wie beim Glas.

## Patentansprüche

1. Verwendung von bioaktiven Glaspartikeln zur Herstellung eines Mittels für eine ästhetische permanente Dentalglaspartikel enthaltende Zahnfüllung, wobei die Zahnfüllung eine permanente Füllung ist, die zusätzlich Dentalglaspartikel enthält, und wobei die eine Hydroxylapatitschicht bildenden Glaspartikel eine Teilchengröße von d₅₀ < 50 µm aufweisen und die Gesamtmenge an Glaspartikeln bis zu 87 Gew.-% der Füllung beträgt.

2. Verwendung nach Anspruch 1, **dadurch gekennzeichnet, dass** der Anteil des bioaktiven Glases in der Materialmatrix höchstens 20 Gew.% beträgt.

3. Verwendung nach Anspruch 1 oder 2, **dadurch gekennzeichnet, dass** der optische Brechungsindex des bioaktiven Glases und/oder des nicht bioaktiven Dentalglases angenähert gleich dem Brechungsindex der umgebenden Matrix ist.

4. Verwendung nach einem der vorhergehenden Ansprüche, **dadurch gekennzeichnet, dass** das nicht bioaktive Dentalglas einen Brechungsindex von n_{d} 1,48-1,65 aufweist.

5. Verwendung nach einem der vorhergehenden Ansprüche, **dadurch gekennzeichnet, dass** das bioaktive Glas und/oder das nicht bioaktive Glas zur Einbindung in eine Harzmatrix silanisiert sind.

6. Verwendung nach einem der vorhergehenden Ansprüche, **dadurch gekennzeichnet, dass** das Glasmaterial 2 bis 10 Gew.% Fluorid enthält.

7. Verwendung nach einem der vorhergehenden Ansprüche, **dadurch gekennzeichnet, dass** dem Zahnfüllmittel Opaker, insbesondere TiO₂, und/oder farbige Pigmente zugemischt sind.

8. Verwendung von bioaktivem Glas nach einem der vorhergehenden Ansprüche, **dadurch gekennzeichnet, dass** das bioaktive Glas und/oder die umgebende Harzmatrix und/oder das nicht bioaktive Dentalglas eine röntgenopake Substanz enthält.

9. Verwendung nach einem der vorhergehenden Ansprüche, **dadurch gekennzeichnet, dass** in dem bioaktiven Glas Ca ganz oder teilweise durch Sr und/oder Ba ersetzt ist.

10. Verwendung von bioaktivem Glas nach einem der vorhergehenden Ansprüche, **dadurch gekennzeichnet, dass** das Füllmittel ein Bindemittel umfassend Acrylat, insbesondere PMMA (Polymethylmethacrylat) oder/und bis-GMA enthält.

11. Verwendung von bioaktives Glas enthaltendem Material nach einem der vorhergehenden Ansprüche, **dadurch gekennzeichnet, dass** das Glas Partikel mit einer durchschnittlichen Korngröße d50 von < 5 µm umfasst.

12. Verwendung nach einem der vorhergehenden Ansprüche, **dadurch gekennzeichnet, dass** das Mittel bioaktives Glas in einem Bindemittel zur Verbindung einer Zahnfüllung mit einem Zahn, in Glas-Ionomer-Zement, in einem Glas-Kunststoff-Composit, in compositverstärkten Glas-Ionomer-Zement und/oder in einem Mittel zur Behandlung der Zahnwurzel, des Zahnhalses und/oder der Zahnkrone enthält.

13. Bindemittel zur Verbindung einer Zahnfüllung mit einem Zahn, **dadurch gekennzeichnet, dass** es bioaktives Glas enthält, das bei Kontakt mit Speichel auf seiner Oberfläche eine Hydroxylapatitschicht ausbildet.

14. Bioaktives Dentalglas, zur Verwendung nach einem der vorhergehenden Ansprüche, **dadurch gekennzeichnet, dass** es Fluorid enthält.

15. Zahnfüllung erhältlich durch die Verwendung nach einem der Ansprüche 1 - 12.

## Claims

1. Use of bioactive glass particles in the preparation of a means for an aesthetical permanent dental filling comprising dental glass particles, the tooth filling being a permanent filling which additionally comprises dental glass particles and the glass particles, which form a hydroxyapatite layer, exhibiting a particle size of d₅₀ < 50 µm and the total amount of glass particles amounting to up to 87% by weight of the filling.

2. Use according to Claim 1, **characterized in that** the proportion of the bioactive glass in the material matrix is at most 20% by weight.

3. Use according to Claim 1 or 2, **characterized in that** the optical refractive index of the bioactive glass and/or of the nonbioactive dental glass is approximately the same as the refractive index of the surrounding matrix.

4. Use according to one of the preceding claims, **characterized in that** the nonbioactive dental glass exhibits a refractive index of n_{d} 1.48-1.65.

5. Use according to one of the preceding claims, **characterized in that** the bioactive glass and/or the nonbioactive glass are silanized for integration in a resin matrix.

6. Use according to one of the preceding claims, **characterized in that** the glass material comprises from 2 to 10% by weight of fluoride.

7. Use according to one of the preceding claims, **characterized in that** opacifiers, in particular TiO₂, and/or coloured pigments are mixed with the dental filling material.

8. Use of bioactive glass according to one of the preceding claims, **characterized in that** the bioactive glass and/or the surrounding resin matrix and/or the nonbioactive dental glass comprises a substance opaque to X-rays.

9. Use according to one of the preceding claims, **characterized in that**, in the bioactive glass, Ca is completely or partially replaced by Sr and/or Ba.

10. Use of bioactive glass according to one of the preceding claims, **characterized in that** the filling material comprises a binder which includes acrylate, in particular PMMA (polymethyl methacrylate) and/or bis-GMA.

11. Use of material comprising bioactive glass according to one of the preceding claims, **characterized in that** the glass comprises particles with an average particle size d₅₀ of < 5 µm.

12. Use according to one of the preceding claims, **characterized in that** the means comprises bioactive glass in a binder for the bonding of a dental filling to a tooth, in glass ionomer cement, in a glass-plastic composite, in composite-reinforced glass ionomer cement and/or in a means for the treatment of dental roots, tooth necks and/or dental crowns.

13. Binder for the bonding of a dental filling to a tooth, **characterized in that** it comprises bioactive glass which forms, on contact with saliva, a hydroxyapatite layer on its surface.

14. Bioactive dental glass for the use according to one of Claims 1-12, **characterized in that** it comprises fluoride.

15. Dental filling obtainable by the use according to one of Claims 1-12.

## Revendications

1. Utilisation de particules de verre bioactives, pour la préparation d'un agent pour un plombage dentaire permanent esthétique contenant des particules de verre dentaire, le plombage dentaire étant un plombage permanent qui contient des particules de verre dentaire, et les particules de verre qui forment une couche d'hydroxyapatite ayant une taille de particule de d₅₀ < 50 µm et la quantité totale des particules de verre constituant jusqu'à 87 % en poids du plombage.

2. Utilisation selon la revendication 1, **caractérisée en ce que** la proportion du verre bioactif dans la matrice de matière est d'au maximum 20 % en poids.

3. Utilisation selon la revendication 1 ou 2, **caractérisée en ce que** l'indice de réfraction optique du verre bioactif et/ou du verre dentaire non bioactif est approximativement égal à l'indice de réfraction de la matrice environnante.

4. Utilisation selon l'une quelconque des revendications précédentes, **caractérisée en ce que** le verre dentaire non bioactif présente un indice de réfraction de n_{d} 1,48-1,65.

5. Utilisation selon l'une quelconque des revendications précédentes, **caractérisée en ce que** le verre bioactif et/ou le verre non bioactif sont silanés pour l'intégration dans une matrice de résine.

6. Utilisation selon l'une quelconque des revendications précédentes, **caractérisée en ce que** le matériau à base de verre contient de 2 à 10 % de fluorure.

7. Utilisation selon l'une quelconque des revendications précédentes, **caractérisée en ce que** des opacifiants, en particulier TiO₂, et/ou des pigments colorés sont incorporés à l'amalgame dentaire.

8. Utilisation de verre bioactif selon l'une quelconque des revendications précédentes, **caractérisée en ce que** le verre bioactif et/ou la matrice de résine environnante et/ou le verre dentaire non bioactif contient/contiennent une substance opaque aux rayons X.

9. Utilisation selon l'une quelconque des revendications précédentes, **caractérisée en ce que** dans le verre bioactif le Ca est totalement ou partiellement remplacé par Sr et/ou Ba.

10. Utilisation de verre bioactif selon l'une quelconque des revendications précédentes, **caractérisée en ce que** l'amalgame contient un liant comprenant un acrylate, en particulier du PMMA [poly(méthacrylate de méthyle)] et/ou du bis-GMA.

11. Utilisation de matériau contenant du verre bioactif selon l'une quelconque des revendications précédentes, **caractérisée en ce que** le verre comprend des particules ayant une taille moyenne de grain d₅₀ de < 5 µm.

12. Utilisation selon l'une quelconque des revendications précédentes, **caractérisée en ce que** le composant contient du verre bioactif dans un liant pour l'assemblage d'un plombage dentaire avec une dent, dans un ciment verre-ionomère, dans un composite verre-matière plastique, dans un ciment ionomère-verre renforcé avec un composite et/ou dans un agent destiné au traitement de la racine dentaire, du collet dentaire et/ou de la couronne dentaire.

13. Liant destiné à l'assemblage d'un plombage dentaire avec une dent, **caractérisé en ce qu'**il contient un verre bioactif qui, au contact avec la salive, forme sur sa surface une couche d'hydroxyapatite.

14. Verre dentaire bioactif, destiné à l'utilisation selon l'une quelconque des revendications 1 - 12, **caractérisé en ce qu'**il contient un fluorure.

15. Plombage dentaire pouvant être obtenu par l'utilisation selon l'une quelconque des revendications 1 - 12.
